# EUROPEAN PATENT APPLICATION

(11) **EP 0 881 290 A2**
(43) Date of publication of application: **02.12.1998**
(21) Application number: 98302907.5
(22) Date of filing: 15.04.1998
(51) Int. Cl.: C12N 15/12, C07K 14/705, C07K 16/18, A61K 38/17, G01N 33/68

(54) **Neurotransmitter transporter HPDDV78**

(30) Priority: 27.05.1997 GB 9710906; 08.12.1997 EP 97309887
(71) Applicant: SMITHKLINE BEECHAM PLC, Brentford, Middlesex TW8 9EP (GB)
(72) Inventor: Brown, Anthony M., Harlow, Essex CM19 5AW (GB); Evans, Joanne R., Harlow, Essex CM19 5AW (GB)
(74) Representative: Crump, Julian Richard John

(57) **Abstract**

HPDDV78 polypeptides and polynucleotides and methods for producing such polypeptides by recombinant techniques are disclosed. Also disclosed are methods for utilizing HPDDV78 polypeptides and polynucleotides in the design of protocols for the treatment of brain hypoxia, trauma, seizures and stroke, among others, and diagnostic assays for such conditions.

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to neurotransmitter transporters family, hereinafter referred to as HPDDV78. The invention also relates to inhibiting or activating the action of such polynucleotides and polypeptides.

### BACKGROUND OF THE INVENTION

Reliable neurotransmission depends on rapid termination of the transmitter's action following post-synaptic activation. In some cases this is achieved by metabolism of the neurotransmitters, as in the case of acetycholine (ACh) and neuropeptides. In many cases, however, including catecholamines, serotonin and some amino acids (e.g. GABA, Glycine and glutamate), the neurotransmitter is efficiently removed into the presynaptic terminal or surrounding glial cells by neurotransmitter transporters, membrane-bound polypeptides located in the plasma membrane.

Recently, cDNAs encoding a number of Na/Cl-dependent neurotransmitter transporters have been described (e.g. serotonin, catecholamine, amino acid (glycine, GABA)). The general structure of this class of transporter is very similar, containing twelve potential transmembrane helices and an external loop with 3-4 glycosylation sites between transmembrane segments 3 and 4. The calculated molecular weights of the transporters is about 70kDa and both their N- and C-terminal portions contain about 40 amino acids and may be located on the cytoplasmic side of the membrane. In GABA and catecholamine transporter subfamilies, the amino acid sequence is about 60-80% identical to other members within a subfamily and about 40% identical to members between two subfamilies (Liu et al., Proc. Natl. Acad. Sci. USA, 89:6639-6643 (1992)). Transporters of amino acids such as glycine and proline share about 40-50% homology with all members of the neurotransmitter transporter superfamily. Sequence homology among the members of the neurotransmitter transporter family give clear indication that they evolved from a common ancestral gene. In addition to these transporters whose endogenous substrates have been identified, there have been described a number of transporters of unknown substrate which show significant amino acid homology with neurotransmitter transporters. One of these so called "orphan" transporters is rB21a (Smith KE et al., FEBS Letts **357** (1995), 86-92), whose localisation in leptomeninges of rat brain suggests a possible role in regulating composition and /or volume of cerebrospinal fluid (CSF) and hence in homeostatic mechanisms to control brain swelling after hypoxia, trauma, seizures or stroke.

Modulation of neurotransmitter uptake enables synaptic transmission to be increased or decreased by altering the levels of neurotransmitter in the synaptic cleft and blockade of uptake mechanisms in this way is an established approach in the treatment of psychiatric illness. Drugs which act by this mechanism include the tricyclic antidepressants, which act on monamine transporters in general, the selective serotonin uptake inhibitors (SSRIs) and the GABA transporter blocker tiagabine (Lesch KP and Bengel D, CNS Drugs 4(1995), 302-322). There remains a need for identification and characterization of further members of neurotransmitter transporters family which can play a role in preventing, ameliorating or correcting dysfunctions or diseases, including, but not limited to, brain hypoxia, trauma, seizures and stroke.

### SUMMARY OF THE INVENTION

In one aspect, the invention relates to HPDDV78 polypeptides and recombinant materials and methods for their production. Another aspect of the invention relates to methods for using such HPDDV78 polypeptides and polynucleotides. Such uses include the treatment of brain hypoxia, trauma, seizures and stroke, among others. In still another aspect, the invention relates to methods to identify agonists and antagonists using the materials provided by the invention, and treating conditions associated with HPDDV78 imbalance with the identified compounds. Yet another aspect of the invention relates to diagnostic assays for detecting diseases associated with inappropriate HPDDV78 activity or levels.

### DESCRIPTION OF THE INVENTION

### Definitions

The following definitions are provided to facilitate understanding of certain terms used frequently herein.

"HPDDV78" refers, among others, generally to a polypeptide having the amino acid sequence set forth in SEQ ID NO:2 or an allelic variant thereof.

"HPDDV78 activity or HPDDV78 polypeptide activity" or "biological activity of the HPDDV78 or HPDDV78 polypeptide" refers to the metabolic or physiologic function of said HPDDV78 including similar activities or improved activities or these activities with decreased undesirable side-effects. Also included are antigenic and immunogenic activities of said HPDDV78.

"HPDDV78 gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NYAcad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., JMolec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to HPDDV78 polypeptides (or HPDDV78 proteins). The HPDDV78 polypeptides include the polypeptide of SEQ ID NO:2; as well as polypeptides comprising the amino acid sequence of SEQ ID NO: 2; and polypeptides comprising the amino acid sequence which have at least 90% identity to that of SEQ ID NO:2 over its entire length, and preferably at least 95% identity to SEQ ID NO: 2. Furthermore, those with at least 97-99% are highly preferred. Also included within HPDDV78 polypeptides are polypeptides having the amino acid sequence which have at least 90% identity to the polypeptide having the amino acid sequence of SEQ ID NO:2 over its entire length, and preferably at least 95% identity to SEQ ID NO:2. Furthermore, those with at least 97-99% are highly preferred. Preferably HPDDV78 polypeptide exhibit at least one biological activity of HPDDV78.

The HPDDV78 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the HPDDV78 polypeptides are also included in the invention. A fragment is a polypeptide having an amino acid sequence that entirely is the same as part, but not all, of the amino acid sequence of the aforementioned HPDDV78 polypeptides. As with HPDDV78 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of HPDDV78 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of HPDDV78 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate HPDDV78 activity, including those with a similar activity or an improved activity, or with a decreased undesirable activity. Also included are those that are antigenic or immunogenic in an animal, especially in a human.

Preferably, all of these polypeptide fragments retain the biological activity of the HPDDV78, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The HPDDV78 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to HPDDV78 polynucleotides. HPDDV78 polynucleotides include isolated polynucleotides which encode the HPDDV78 polypeptides and fragments, and polynucleotides closely related thereto. More specifically, HPDDV78 polynucleotide of the invention include a polynucleotide comprising the nucleotide sequence contained in SEQ ID NO:1 encoding a HPDDV78 polypeptide of SEQ ID NO: 2, and polynucleotides having the particular sequence of SEQ ID NO:1. HPDDV78 polynucleotides further include a polynucleotide comprising a nucleotide sequence that has at least 90% identity over its entire length to a nucleotide sequence encoding the HPDDV78 polypeptide of SEQ ID NO:2, and a polynucleotide comprising a nucleotide sequence that is at least 90% identical to that of SEQ ID NO:1 over its entire length. In this regard, polynucleotides at least 95% are preferred. Furthermore, those with at least 97% are highly preferred and those with at least 98-99% are most highly preferred, with at least 99% being the most preferred. Also included under HPDDV78 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such HPDDV78 polynucleotides.

HPDDV78 of the invention is structurally related to other proteins of the neurotransmitter transporters family, as shown by the results of sequencing the cDNA encoding human HPDDV78. The cDNA sequence of SEQ ID NO:1 contains an open reading frame (nucleotide number 167 to 2107) encoding a polypeptide of 647 amino acids of SEQ ID NO:2. Amino acid sequence of SEQ ID NO:2 has about 89% identity (using BLASTX) in 603 amino acid residues with with rat neurotransmitter transporter rB21a (Smith KE et al., FEBS Letts **357** (1995), 86-92). Nucleotide sequence of SEQ ID NO:1 has about 87% identity (using BLASTN) in 1803 nucleotide residues with with rat neurotransmitter transporter rB21a (Smith KE et al., FEBS Letts **357** (1995), 86-92).

The present invention also relates to partial or other polynucleotide and polypeptide sequences which were first identified prior to the determination of the corresponding full length sequences of SEQ ID NO: 1 and SEQ ID NO:2.

Accordingly, in a further aspect, the present invention provides for an isolated polynucleotide comprising:
(a) a nucleotide sequence which has at least 85% identity, preferably at least 90% identity, more preferably at least 95% identity, most preferably at least 97-99% identity to SEQ ID NO:3 over the entire length of SEQ ID NO:3;
(b) a nucleotide sequence which has at least 85% identity, preferably at least 90% identity, more preferably at least 95% identity, even more preferably at least 97-99% identity, to SEQ ID NO:3 over the entire length of SEQ ID NO:3;
(c) the polynucleotide of SEQ ID NO:3; or
(d) a nucleotide sequence encoding a polypeptide which has at least 85% identity, preferably at 90% identity, more preferably at least 95% identity, yet more preferably at least 97-99% identity, to the amino acid sequence of SEQ ID NO:4, over the entire length of SEQ ID NO:4; as well as the polynucleotide of SEQ ID NO:3.

The present invention further provides for a polypeptide which:
(a) comprises an amino acid sequence which has at least 85% identity, preferably at least 90% identity, more preferably at least 95% identity, most preferably at least 97-99% identity, to that of SEQ ID NO:4 over the entire length of SEQ ID NO:4;
(b) has an amino acid sequence which is at least 85% identity, preferably at least 90% identity, more preferably at least 95% identity, most preferably at least 97-99% identity, to the amino acid sequence of SEQ ID NO:4 over the entire length of SEQ ID NO:4;
(c) comprises the amino acid of SEQ ID NO:4; and
(d) is the polypeptide of SEQ ID NO:4;
as well as polypeptides encoded by a polynucleotide comprising the sequence contained in SEQ ID NO:3.

The nucleotide sequence of SEQ ID NO:3 and the peptide sequence encoded thereby are derived from EST (Expressed Sequence Tag) sequences. It is recognised by those skilled in the art that there will inevitably be some nucleotide sequence reading errors in EST sequences (see Adams, M.D. *et al,* Nature 377 (supp) 3, 1995). Accordingly, the nucleotide sequence of SEQ ID NO:3 and the peptide sequence encoded therefrom are therefore subjec to the same inherent limitations in sequence accuracy. Furthermore, the peptide sequence encoded by SEQ ID NO:3 comprises a region of identity or close homology and/or close structural similarity (for example a conservative amino acid difference) with the closest homologous or structurally similar protein.

The cDNA sequence of SEQ ID NO:3 contains an open reading frame (nucleotide number 1 to 577) encoding a polypeptide of 192 amino acids of SEQ ID NO:4. Amino acid sequence of SEQ ID NO:4 has about 84% identity (using BLASTX) in 191 amino acid residues with rat neurotransmitter transporter rB21a (Smith KE et al., FEBS Letts **357** (1995), 86-92). Nucleotide sequence of SEQ ID NO:3 has about 82% identity (using BLASTN) in 592 nucleotide residues with rat neurotransmitter transporter rB21a. (Smith KE et al., FEBS Letts **357** (1995), 86-92).

One polynucleotide of the present invention encoding HPDDV78 may be obtained using standard cloning and screening, from a cDNA library derived from mRNA in cells of human pancreatic carcinoma using the expressed sequence tag (EST) analysis (Adams, M.D., *et al. Science* (1991) 252:1651-1656; Adams, M.D. *et al., Nature,* (1992) *355:632-634;* Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding HPDDV78 polypeptide of SEQ ID NO:2 may be identical to the polypeptide encoding sequence contained in SEQ ID NO: I(nucleotide number 167 to 2107 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of HPDDV78 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexahistidine peptide, as provided in the pQE vector (Qiagen, Inc.) and described in Gentz *et al., Proc Natl Acad Sci USA* (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA.

Further preferred embodiments are polynucleotides encoding HPDDV78 variants comprising the amino acid sequence of HPDDV78 polypeptide of Table 2 (SEQ ID NO:2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO: 1 or a fragment thereof (including that of SEQ ID NO:3), may be used as hybridization probes for cDNA and genomic DNA, to isolate full-length cDNAs and genomic clones encoding HPDDV78 polypeptide and to isolate cDNA and genomic clones of other genes (including genes encoding homologs and orthologs from species other than human) that have a high sequence similarity to the HPDDV78 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding HPDDV78 polypeptide, including homologs and orthologs from species other than human, comprises the steps of screening an appropriate library under stingent hybridization conditions with a labeled probe having the SEQ ID NO: 1 or a fragment thereof (including that of SEQ ID NO: 3), and isolating full-length cDNA and genomic clones containing said polynucleotide sequence. Such hybridization techniques are well known to those of skill in the art. Thus in another aspect, HPDDV78 polynucleotides of the present invention further include a nucleotide sequence comprising a nucleotide sequence that hybridize under stringent condition to a nucleotide sequence having SEQ ID NO: 1 or a fragment thereof (including that of SEQ ID NO:3). Also included with HPDDV78 polypeptides are polypeptide comprising amino acid sequence encoded by nucleotide sequence obtained by the above hybridization condition. Stringent hybridization conditions are as defined above or, alternatively, conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*)*.*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the HPDDV78 polypeptide is to be expressed for use in screening assays, generally, it is preferred that the polypeptide be produced at the surface of the cell. In this event, the cells may be harvested prior to use in the screening assay. If HPDDV78 polypeptide is secreted into the medium, the medium can be recovered in order to recover and purify the polypeptide; if produced intracellularly, the cells must first be lysed before the polypeptide is recovered. HPDDV78 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of HPDDV78 polynucleotides for use as diagnostic reagents. Detection of a mutated form of HPDDV78 gene associated with a dysfunction will provide a diagnostic tool that can add to or define a diagnosis of a disease or susceptibility to a disease which results from under-expression, over-expression or altered expression of HPDDV78. Individuals carrying mutations in the HPDDV78 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HPDDV78 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising HPDDV78 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to brain hypoxia, trauma, seizures and stroke through detection of mutation in the HPDDV78 gene by the methods described.

In addition, brain hypoxia, trauma, seizures and stroke, can be diagnosed by methods comprising determining from a sample derived from a subject an abnormally decreased or increased level of HPDDV78 polypeptide or HPDDV78 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an HPDDV78 polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly brain hypoxia, trauma, seizures and stroke, which comprises:
(a) a HPDDV78 polynucleotide, preferably the nucleotide sequence of SEQ ID NO: 1, or a fragment thereof;
(b) a nucleotide sequence complementary to that of (a);
(c) a HPDDV78 polypeptide, preferably the polypeptide of SEQ ID NO: 2, or a fragment thereof; or
(d) an antibody to a HPDDV78 polypeptide, preferably to the polypeptide of SEQ ID NO: 2.
It will be appreciated that in any such kit, (a), (b), (c) or (d) may comprise a substantial component.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the HPDDV78 polypeptides. The term "immunospecific" means that the antibodies have substantiall greater affinity for the polypeptides of the invention than their affinity for other related polypeptides in the prior art.

Antibodies generated against the HPDDV78 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against HPDDV78 polypeptides may also be employed to treat brain hypoxia, trauma, seizures and stroke, among others.

### Vaccines

Another aspect of the invention relates to a method for inducing an immunological response in a mammal which comprises inoculating the mammal with HPDDV78 polypeptide, or a fragment thereof, adequate to produce antibody and/or T cell immune response to protect said animal from brain hypoxia, trauma, seizures and stroke, among others. Yet another aspect of the invention relates to a method of inducing immunological response in a mammal which comprises, delivering HPDDV78 polypeptide via a vector directing expression of HPDDV78 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a HPDDV78 polypeptide wherein the composition comprises a HPDDV78 polypeptide or HPDDV78 gene. The vaccine formulation may further comprise a suitable carrier. Since HPDDV78 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The HPDDV78 polypeptide of the present invention may be employed in a screening process for compounds which activate (agonists) or inhibit activation of (antagonists, or otherwise called inhibitors) the HPDDV78 polypeptide of the present invention. Thus, polypeptides of the invention may also be used to assess identify agonist or antagonists from, for example, cells, cell-free preparations, chemical libraries, and natural product mixtures. These agonists or antagonists may be natural or modified substrates, ligands, enzymes, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

HPDDV78 polypeptides are responsible for many biological functions, including many pathologies. Accordingly, it is desirous to find compounds and drugs which stimulate HPDDV78 polypeptide on the one hand and which can inhibit the function of HPDDV78 polypeptide on the other hand. In general, agonists are employed for therapeutic and prophylactic purposes for such conditions as brain hypoxia, trauma, seizures and stroke. Antagonists may be employed for a variety of therapeutic and prophylactic purposes for such conditions as brain hypoxia, trauma, seizures and stroke.

In general, such screening procedures may involve using appropriate cells which express the HPDDV78 polypeptide or respond to HPDDV78 polypeptide of the present invention. Such cells include cells from mammals, yeast, *Drosophila* or *E. coli.* Cells which express the HPDDV78 polypeptide (or cell membrane containing the expressed polypeptide) or respond to HPDDV78 polypeptide are then contacted with a test compound to observe binding, or stimulation or inhibition of a functional response. The ability of the cells which were contacted with the candidate compounds is compared with the same cells which were not contacted for HPDDV78 activity.

As an example, activity of the transporter may be determined in cells in culture through use of a radiolabelled substrate. After exposure of the cells to the substrate (added to the culture medium) for a defined length of time, the cells are washed, their contents extracted by treatment with acid or alkali and the cellular content of radiolabel determined by scintillation spectrometry. In this way, maximal activity, substrate concentration required for half maximal activity (Km) and the potency of competing agents (e.g. small molecules) can be determined by established methods (see e.g. Clark JA and Amara S, Mol. Pharmacol. **46** (1994), 550-557).

The assays may simply test binding of a candidate compound wherein adherence to the cells bearing the HPDDV78 polypeptide is detected by means of a label directly or indirectly associated with the candidate compound or in an assay involving competition with a labeled competitor. Further, these assays may test whether the candidate compound results in a signal generated by activation of the HPDDV78 polypeptide, using detection systems appropriate to the cells bearing the HPDDV78 polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a HPDDV78 polypeptide to form a mixture, measuring HPDDV78 activity in the mixture, and comparing the HPDDV78 activity of the mixture to a standard.

The HPDDV78 cDNA, protein and antibodies to the protein may also be used to configure assays for detecting the effect of added compounds on the production of HPDDV78 mRNA and protein in cells. For example, an ELISA may be constructed for measuring secreted or cell associated levels of HPDDV78 protein using monoclonal and polyclonal antibodies by standard methods known in the art, and this can be used to discover agents which may inhibit or enhance the production of HPDDV78 (also called antagonist or agonist, respectively) from suitably manipulated cells or tissues.

The HPDDV78 protein may be used to identify membrane bound or soluble receptors, if any, through standard receptor binding techniques known in the art. These include, but are not limited to, ligand binding and crosslinking assays in which the HPDDV78 is labeled with a radioactive isotope (eg 1251), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of HPDDV78 which compete with the binding of HPDDV78 to its receptors, if any. Standard methods for conducting screening assays are well understood in the art.

Examples of potential HPDDV78 polypeptide antagonists include antibodies or, in some cases, oligonucleotides or proteins which are closely related to the ligands, substrates, enzymes, receptors, etc., as the case may be, of the HPDDV78 polypeptide, e.g., a fragment of the ligands, substrates, enzymes, receptors, etc.; or small molecules which bind to the polypeptide of the present invention but do not elicit a response, so that the activity of the polypeptide is prevented.

Thus in another aspect, the present invention relates to a screening kit for identifying agonists, antagonists, ligands, receptors, substrates, enzymes, etc. for HPDDV78 polypeptides; or compounds which decrease or enhance the production of HPDDV78 polypeptides, which comprises:
(a) a HPDDV78 polypeptide, preferably that of SEQ ID NO:2;
(b) a recombinant cell expressing a HPDDV78 polypeptide, preferably that of SEQ ID NO:2;
(c) a cell membrane expressing a HPDDV78 polypeptide; preferably that of SEQ ID NO: 2; or
(d) antibody to a HPDDV78 polypeptide, preferably that of SEQ ID NO: 2.
It will be appreciated that in any such kit, (a), (b), (c) or (d) may comprise a substantial component.

### Prophylactic and Therapeutic Methods

This invention provides methods of treating abnormal conditions such as, brain hypoxia, trauma, seizures and stroke, related to both an excess of and insufficient amounts of HPDDV78 polypeptide activity.

If the activity of HPDDV78 polypeptide is in excess, several approaches are available. One approach comprises administering to a subject an inhibitor compound (antagonist) as hereinabove described along with a pharmaceutically acceptable carrier in an amount effective to inhibit the function of the HPDDV78 polypeptide, such as, for example, by blocking the binding of ligands, substrates, enzymes, receptors, etc., or by inhibiting a second signal, and thereby alleviating the abnormal condition. In another approach, soluble forms of HPDDV78 polypeptides still capable of binding the ligand, substrate, enzymes, receptors, etc. in competition with endogenous HPDDV78 polypeptide may be administered. Typical embodiments of such competitors comprise fragments of the HPDDV78 polypeptide.

In another approach, soluble forms of HPDDV78 polypeptides still capable of binding the ligand in competition with endogenous HPDDV78 polypeptide may be administered. Typical embodiments of such competitors comprise fragments of the HPDDV78 polypeptide.

In still another approach, expression of the gene encoding endogenous HPDDV78 polypeptide can be inhibited using expression blocking techniques. Known such techniques involve the use of antisense sequences, either internally generated or separately administered. See, for example, O'Connor, *JNeurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of HPDDV78 and its activity, several approaches are also available. One approach comprises administering to a subject a therapeutically effective amount of a compound which activates HPDDV78 polypeptide, i.e., an agonist as described above, in combination with a pharmaceutically acceptable carrier, to thereby alleviate the abnormal condition. Alternatively, gene therapy may be employed to effect the endogenous production of HPDDV78 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of HPDDV78 polypeptides in combination with a suitable pharmaceutical carrier.

### Formulation and Administration

Peptides, such as the soluble form of HPDDV78 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

All publications, including but not limited to patents and patent applications, cited in this specification are herein incorporated by reference as if each individual publication were specifically and individually indicated to be incorporated by reference herein as though fully set forth.

SEQ ID NO: 1ª

1 AACTCAGTTG GAGTCAAGAA AAAAGCTAAG GAAATCTTTT AGAAAAATAG
51 AGCAAAAATG AGGAGGAAAG TTTTAAAAAA ACAAGAGAGA AAAACATATT
101 TTTAAAAGAC AGGACATTTA CCTAAATGAG TTGAAAACTC ATGTCCACAC
151 AAAAACCTGC ACACAGATGT TTATAACAGT TTTATTCATA GCTGGCAAAA
201 CTTGGAAGCA ACCAAGATGT TCTTCTGGAG GTGAATGGAT AAATAAACTG
251 TGGCACATCC AGACAATGGA ATGCACTGAG ACTGCCGTGC CGGACCCCCG
301 AGCCGGAGCC GAGCGCGCCG AGGCCGGGGC CATGGAGAAA GCGCGGCCGC
351 TGTGGGCCAA CTCGCTACAG TTCGTGTTCG CCTGCATCTC GTACGCCGTG
401 GGCCTGGGCA ACGTGTGGCG ATTCCCGTAC CTGTGCCAGA TGTACGGCGG
451 AGGTAGTTTC CTGGTCCCCT ACATCATCAT GCTTATCGTG GAGGGAATGC
501 CGCTCTTGTA CCTGGAACTG GCTGTGGGGC AGCGCATGCG GCAGGGCAGC
551 ATCGGCGCCT GGAGGACCAT CAGCCCGTAC CTCAGTGGTG TCGGGGTCGC
601 CAGCGTGGTG GTCTCTTTCT TCCTCTCCAT GTACTACAAC GTCATCAACG
651 CCTGGGCCTT CTGGTACCTC TTCCACTCCT TCCAGGATCC CCTGCCGTGG
701 TCTGTCTGCC CACTGAATGG TAACCACACG GGCTGCGATG AGGAGTGCGA
751 GAAGGCGTCC TCCACACAGT ACTTCTGGTA CAGGAAAACC CTCAATATCT
801 CGCCGTCCCT CCAGGAGAAC GGGGGTGTGC AGTGGGAGCC GGCGCTGTGC
851 CTCCTCCTGG CCTGGCTGGT GGTGTACCTG TGCATCCTGC GTGGCACCGA
901 GTCCACTGGC AAGGTGGTGT ATTTCACGGC GTCACTGCCC TATTGCGTGC
951 TCATCATCTA CCTCATCAGG GGCCTCACGC TCCACGGAGC CACCAATGGC
1001 CTCATGTACA TGTTCACTCC CAAGATAGAG CAGCTGGCCA ACCCCAAGGC
1051 CTGGATCAAT GCAGCCACCC AGATTTTCTT CTCACTTGGC CTGGGCTTCG
1101 GCAGCCTGAT CGCCTTCGCC AGCTACAATG AGCCATCCAA CAACTGCCAG
1151 AAGCACGCCA TCATCGTGTC CCTCATCAAC AGCTTCACCT CCATATTTGC
1201 CAGCATTGTC ACCTTCTCCA TCTATGGCTT CAAGGCCACC TTCAATTATG
1251 AAAACTGCTT GAAGAAGGTG AGTCTGCTGC TGACCAACAC TTTTGACCTT
1301 GAAGATGGCT TTTTGACAGC CAGCAACCTG GAGCAGGTGA AGGGCTACCT
1351 CGCATCTGCC TACCCAAGCA AATACAGCGA GATGTTCCCG CAAATCAAAA
1401 ACTGCAGCTT GGAATCGGAG CTAGACACGG CCGTCCAGGG CACTGGCCTG
1451 GCATTCATCG TCTACACAGA GGCCATTAAA AACATGGAGG TGTCCCAGCT
1501 GTGGTCGGTG CTCTACTTCT TCATGCTGCT GATGCTGGGC ATTGGGAGCA
1551 TGCTGGGGAA CACAGCGGCC ATCCTCACCC CTCTGACAGA CAGCAAGATC
1601 ATCTCCAGCC ACCTGCCCAA GGAGGCCATC TCAGGTCTGG TGTGCCTTGT
1651 CAACTGTGCC ATTGGCATGG TGTTCACGAT GGAGGCTGGG AACTACTGGT
1701 TTGACATATT CAACGACTAC GCGGCCACAC TGTCCCTGCT GCTCATCGTG
1751 CTGGTGGAGA CGATTGCCGT GTGCTACGTG TACGGGCTGA GGAGATTTGA
1801 AAGTGACCTT AAGGCCATGA CCGGCCGAGC TGTGAGCTGG TACTGGAAGG
1851 TGATGTGGGC TGGCGTAAGC CCACTGCTGA TTGTCAGCCT CTTTGTCTTC
1901 TACCTGAGCG ACTACATCCT CACGGGGACC CTGAAGTATC AAGCCTGGGA
1951 CGCCTCCCAG GGCCAGCTCG TGACCAAAGA TTACCCGGCC TATGCACTGG
2001 CTGTCATCGG GCTGCTTGTG GCCTCCTCCA CCATGTGCAT CCCCCTGGCG
2051 GCCCTGGGGA CTTTTGTTCA GCGTCGCCTC AAGAGGGGAG ACGCAGACCC
2101 CGTGGCCTGA GATGTGGGCT TCCCAGCCGC TCACGGTTTT ACAGATACTA
2151 TTTACAGGCG GAAACTCCTC GGCTGCTTTT TCAAATGCTT AAGCCAGGAG
2201 TGCTCAGCCC ATCAACTTCC TGAGTGTCTA AAGAAGATGA GGAAGGTGTG
2251 CAGGAAGAAA ACTCCCTTGG GAGAACGCAC ACCCTCCCGT GGTGGCTGTT
2301 CCTCCCTGTC ACCTGCCTCC TCATCATGGA AGGGGGTGGG CTATGAAAGC
2351 CGGTCTCAAA GATAACTGCA TCCTTCATTC CAGGAAAGCC CTAGAATTAG
2401 GGCACATTGC AAACTGAAAT ATGACTATAA TTCTTATGGG ACCAAATTTA
2451 AGCAATTTTT GTTTTTGGCT GAAGAGACAC CAAAATATTA GAGGACAAAT
2501 ATTTTTAGAT CCATTTAAGG AGTTTTGAAG TGCCTAAGAT GACCTATTTG
2551 TCAGTGGTGC AAAATTAATT CTCTTCTTTT TTGAGTTGTA GTGAATATGC
2601 AATTTCTGTG TTCCCCTTCC ACCCTTTAAA TCTTAGGATG ACAAGTTATA
2651 AAGAAAGAAG ATCTTTGTCT GGGACCCCCA AAGGGATCCT TTCTCTAAGG
2701 TCTCTGACGG TGGGTCCAGG ACCAGACCTC TCTACAAAAA ATTGCCCCAA

2751 CTACAGTTTG CAACCCCAAA CCACATTAGA ATCTGTGCAG ACATCCCTCC
2801 GTGGTGTGTG TCTTGGTGCA TTGGAAAAGG AGTCAGGAGC CACTGTGAGG
2851 TGAGAATGAA AGTGGATCTC AGCTGGGCAC GGTGGCTCAC GCCTGTAATC
2901 CTAGCACCTT GGGGGTCAAG GTGGGTGGAT CACTTGAGGT CAGGAGTTTG
2951 AGGCCAGCCT GGCCAACATG GCGAAACCCC ATTTCTACTA AAAATACAAA
3001 AAATTAGCTG GGAGTGGTGG CATAGGCCTG TAATTCCAGC TACTCTGGCT
3051 GCTGAGGCAC AAGAATCATT TGAACCTAGG AGGTGGCGGT TGCTGTGAGC
3101 CAAGATCATG CCACTCCACT CCAGTCTGGG CGACAGAGCA AGACTCTGAC
3151 TCAAAAAAAA AAAAAAAAAA

a A nucleotide sequence of a human HPDDV78..

SEQ ID NO: 2^b

1 MFITVLFIAG KTWKQPRCSS GGEWINKLWH IQTMECTETA VPDPRAGAER
51 AEAGAMEKAR PLWANSLQFV FACISYAVGL GNVWRFPYLC QMYGGGSFLV
101 PYIIMLIVEG MPLLYLELAV GQRMRQGSIG AWRTISPYLS GVGVASVVVS
151 FFLSMYYNVI NAWAFWYLFH SFQDPLPWSV CPLNGNHTGC DEECEKASST
201 QYFWYRKTLN ISPSLQENGG VQWEPALCLL LAWLVVYLCI LRGTESTGKV
251 VYFTASLPYC VLIIYLIRGL TLHGATNGLM YMFTPKIEQL ANPKAWINAA
301 TQIFFSLGLG FGSLIAFASY NEPSNNCQKH AIIVSLINSF TSIFASIVTF
351 SIYGFKATFN YENCLKKVSL LLTNTFDLED GFLTASNLEQ VKGYLASAYP
401 SKYSEMFPQI KNCSLESELD TAVQGTGLAF IVYTEAIKNM EVSQLWSVLY
451 FFMLLMLGIG SMLGNTAAIL TPLTDSKIIS SHLPKEAISG LVCLVNCAIG
501 MVFTMEAGNY WFDIFNDYAA TLSLLLIVLV ETIAVCYVYG LRRFESDLKA
551 MTGRAVSWYW KVMWAGVSPL LIVSLFVFYL SDYILTGTLK YQAWDASQGQ
601 LVTKDYPAYA LAVIGLLVAS STMCIPLAAL GTFVQRRLKR GDADPVA

^b An amino acid sequence of a human HPDDV78..

SEQ ID NO: 3^c

1 GCTGGGCATTGGGAGCATGCTGGGGAACACAGGnGCCATCCTCACCCCTCTGACAGACAG 60
. . . . . .
61 CAAGATCATCTCCAGCCACCTGCCCAAGGAGGCCATCTCAGGTCTGGTGTGCCTTGTCAA 120
. . . . . .
121 CTGTGCCATTGGCATGGTGTTCACGATGGAGGCTGGGAACTACTGGTTTGACATATTCAA 180
. . . . . .
181 CGACTACGCGGCCACACTGTCCCTGCTGCTCATCGTGCTGGTGGAGACGATTGCCGTGTG 240
. . . . . .
241 CTACGTGTACGGGCTGAGGAGATTTGAAAGTGACCTTAAGGCCATGACCGGCCGAGCTGT 300
. . . . . .
301 GAGCTGGTACTGGAAGGtGATGTGGCTGGCGTAAaCCACTGCaTGATTGTAAGCCTCTT 360
. . . . . .
361 TGTcTTCTACCTGAGCGACTACATCCTCACGGGGACCCTGAAGTATCAAGCCTGGGACGC 420
. . . . . .
421 CTCCCAGGGCCAGCTCGTGACCAAAGATTACCCGGCCTATGCACTGGCTGTcATCGGGCT 480

481 GCTTGTGGCCTCCTCCACCATGTGCATCCCCCTGGCGGCCCTGGGGACTTTTGTTCAGCG 540

541 TCGCCTCAAGAGGGGAGACGCAGACCCCGTGGCCTGAGATGTGGGCTTCCCAGCCGCTCA 600

601 CGGTTTTACAGATACTATTTACAGGCGGAAACTCCTCGGCTGCTTTTTCAAATGCTTAAG 660

661 CCAGGAGTGCTCAGCCCATCAACTTCCTGAGTGTCTAAAGAAGATGAGGAAGGTGTGCA 719

c A partial nucleotide sequence of a human HPDDV78.

SEQ ID NO: 4.d
Leu Gly Ile Gly Ser Met Leu Gly Asn Thr Gly Ala Ile Leu Thr
                5                   10                  15
Pro Leu Thr Asp Ser Lys Ile Ile Ser Ser His Leu Pro Lys Glu
                20                  25                  30
Ala Ile Ser Gly Leu Val Cys Leu Val Asn Cys Ala Ile Gly Met
                35                  40                  45
Val Phe Thr Met Glu Ala Gly Asn Tyr Trp Phe Asp Ile Phe Asn
                50                  55                  60
Asp Tyr Ala Ala Thr Leu Ser Leu Leu Leu Ile Val Leu Val Glu
                65                  70                  75
Thr Ile Ala Val Cys Tyr Val Tyr Gly Leu Arg Arg Phe Glu Ser
                80                  85                  90
Asp Leu Lys Ala Met Thr Gly Arg Ala Val Ser Trp Tyr Trp Lys
                95                  100                 105
Val Met Trp Ala Gly Val Asn His Cys Met Ile Val Ser Leu Phe
                110                 115                 120
Val Phe Tyr Leu Ser Asp Tyr Ile Leu Thr Gly Thr Leu Lys Tyr
                125                 130                 135
Gln Ala Trp Asp Ala Ser Gln Gly Gln Leu Val Thr Lys Asp Tyr
                140                 145                 150
Pro Ala Tyr Ala Leu Ala Val Ile Gly Leu Leu Val Ala Ser Ser
                155                 160                 165
Thr Met Cys Ile Pro Leu Ala Ala Leu Gly Thr Phe Val Gln Arg
                170                 175                 180
Arg Leu Lys Arg Gly Asp Ala Asp Pro Val Ala
                185                 190

d A partial amino acid sequence of a human HPDDV78.

## Claims

**1.** An isolated polynucleotide comprising a nucleotide sequence that has at least 90% identity over its entire length to a nucleotide sequence encoding the HPDDV78 polypeptide of SEQ ID NO:2; or a nucleotide sequence complementary to said isolated polynucleotide.

**2.** The polynucleotide of claim 1 wherein said polynucleotide comprises the nucleotide sequence contained in SEQ ID NO: 1 encoding the HPDDV78 polypeptide of SEQ ID NO2.

**3.** An isolated polynucleotide comprising a nucleotide sequence that has at least 90% identity with SEQ ID NO: 1 over its entire length.

**4.** The polynucleotide of claim 3 which is polynucleotide of SEQ ID NO: 1.

**5.** A DNA or RNA molecule comprising an expression system, wherein said expression system is capable of producing a HPDDV78 polypeptide comprising an amino acid sequence, which has at least 90% identity with the polypeptide of SEQ ID NO:2 when said expression system is present in a compatible host cell.

**6.** A host cell comprising the expression system of claim 5.

**7.** A process for producing a HPDDV78 polypeptide comprising culturing a host of claim 6 under conditions sufficient for the production of said polypeptide and recovering the polypeptide from the culture.

**8.** A process for producing a cell which produces a HPDDV78 polypeptide thereof comprising transforming or transfecting a host cell with the expression system of claim 5 such that the host cell, under appropriate culture conditions, produces a HPDDV78 polypeptide.

**9.** A HPDDV78 polypeptide comprising an amino acid sequence which is at least 90% identical to the amino acid sequence of SEQ ID NO:2 over its entire length.

**10.** The polypeptide of claim 9 which comprises the amino acid sequence of SEQ ID NO:2.

**11.** An antibody immunospecific for the HPDDV78 polypeptide of claim 9.

**12.** A method for the treatment of a subject in need of enhanced activity or expression of HPDDV78 polypeptide of claim 9 comprising:
(a) administering to the subject a therapeutically effective amount of an agonist to said polypeptide; and/or
(b) providing to the subject an isolated polynucleotide comprising a nucleotide sequence that has at least 90% identity to a nucleotide sequence encoding the HPDDV78 polypeptide of SEQ ID NO:2 over its entire length; or a nucleotide sequence complementary to said nucleotide sequence in a form so as to effect production of said polypeptide activity *in vivo.*

**13.** A method for the treatment of a subject having need to inhibit activity or expression of HPDDV78 polypeptide of claim 9 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate, or receptor.

**14.** A process for diagnosing a disease or a susceptibility to a disease in a subject related to expression or activity of HPDDV78 polypeptide of claim 9 in a subject comprising:
(a) determining the presence or absence of a mutation in the nucleotide sequence encoding said HPDDV78 polypeptide in the genome of said subject; and/or
(b) analyzing for the presence or amount of the HPDDV78 polypeptide expression in a sample derived from said subject.

**15.** A method for identifying compounds which inhibit (antagonize) or agonize the HPDDV78 polypeptide of claim 9 which comprises:
(a) contacting a candidate compound with cells which express the HPDDV78 polypeptide (or cell membrane expressing HPDDV78 polypeptide) or respond to HPDDV78 polypeptide; and
(b) observing the binding, or stimulation or inhibition of a functional response; or comparing the ability of the cells (or cell membrane) which were contacted with the candidate compounds with the same cells which were not contacted for HPDDV78 polypeptide activity.

**16.** An agonist or an antagonist identified by the method of claim 15.

**17.** A recombinant host cell produced by a method of Claim 8 or a membrane thereof expressing a HPDDV78 polypeptide.

**19.** An isolated polynucleotide selected form the group consisting of:
(a) an isolated polynucleotide comprising a nucleotide sequence which has at least 85% identity to SEQ ID NO:3 over the entire length of SEQ ID NO:3;
(b) an isolated polynucleotide comprising the polynucleotide of SEQ ID NO:3;
(c) the polynucleotide of SEQ ID NO:3; or
(d) an isolated polynucleotide comprising a nucleotide sequence encoding a polypeptide which has at least 85% identity to the amino acid sequence of SEQ ID NO:4, over the entire length of SEQ ID NO:4.

**20.** A polypeptide selected from the group consisting of:
(a) comprises an amino acid sequence which has at least 85% identity to that of SEQ ID NO:4 over the entire length of SEQ ID NO:4;
(b) has an amino acid sequence which is at least 85% identity to the amino acid sequence of SEQ ID NO:4 over the entire length of SEQ ID NO:4;
(c) comprises the amino acid of SEQ ID NO:4; and
(d) is the polypeptide of SEQ ID NO:4;
(e) a polypeptide which is encoded by a polynucleotide comprising the sequence contained in SEQ ID NO:3.


### Annex to the description

SEQUENCE LISTING

(1) GENERAL INFORMATION

(i) APPLICANT: SB plc

(ii) TITLE OF THE INVENTION: Novel Compound

(iii) NUMBER OF SEQUENCES: 4

(iv) CORRESPONDENCE ADDRESS:
(A) ADDRESSEE: Smith Kline Beecham P.L.C.
(B) STREET:
(C) CITY:
(D) STATE:
(E) COUNTRY:
(F) ZIP:

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: Connell Anthony C
(B) REGISTRATION NUMBER:
(C) REFERENCE/DOCKET NUMBER: GH30309

(ix) TELECOMMUNICATION INFORMATION:
(A) TELEPHONE:
(B) TELEFAX:
(C) TELEX:

(2) INFORMATION FOR SEQ ID NO:1:

(i) SEQUENCE CHARACTERISTICS:
(A) LENGTH: 3169 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

AACTCAGTTG GAGTCAAGAA AAAAGCTAAG GAAATCTTTT AGAAAAATAG AGCAAAAATG 60
AGGAGGAAAG TTTTAAAAAA ACAAGAGAGA AAAACATATT TTTAAAAGAC AGGACATTTA 120
CCTAAATGAG TTGAAAACTC ATGTCCACAC AAAAACCTGC ACACAGATGT TTATAACAGT 180
TTTATTCATA GCTGGCAAAA CTTGGAAGCA ACCAAGATGT TCTTCTGGAG GTGAATGGAT 240
AAATAAACTG TGGCACATCC AGACAATGGA ATGCACTGAG ACTGCCGTGC CGGACCCCCG 300
AGCCGGAGCC GAGCGCGCCG AGGCCGGGGC CATGGAGAAA GCGCGGCCGC TGTGGGCCAA 360
CTCGCTACAG TTCGTGTTCG CCTGCATCTC GTACGCCGTG GGCCTGGGCA ACGTGTGGCG 420
ATTCCCGTAC CTGTGCCAGA TGTACGGCGG AGGTAGTTTC CTGGTCCCCT ACATCATCAT 480
GCTTATCGTG GAGGGAATGC CGCTCTTGTA CCTGGAACTG GCTGTGGGGC AGCGCATGCG 540
GCAGGGCAGC ATCGGCGCCT GGAGGACCAT CAGCCCGTAC CTCAGTGGTG TCGGGGTCGC 600
CAGCGTGGTG GTCTCTTTCT TCCTCTCCAT GTACTACAAC GTCATCAACG CCTGGGCCTT 660
CTGGTACCTC TTCCACTCCT TCCAGGATCC CCTGCCGTGG TCTGTCTGCC CACTGAATGG 720
TAACCACACG GGCTGCGATG AGGAGTGCGA GAAGGCGTCC TCCACACAGT ACTTCTGGTA 780
CAGGAAAACC CTCAATATCT CGCCGTCCCT CCAGGAGAAC GGGGGTGTGC AGTGGGAGCC 840
GGCGCTGTGC CTCCTCCTGG CCTGGCTGGT GGTGTACCTG TGCATCCTGC GTGGCACCGA 900
GTCCACTGGC AAGGTGGTGT ATTTCACGGC GTCACTGCCC TATTGCGTGC TCATCATCTA 960
CCTCATCAGG GGCCTCACGC TCCACGGAGC CACCAATGGC CTCATGTACA TGTTCACTCC 1020
CAAGATAGAG CAGCTGGCCA ACCCCAAGGC CTGGATCAAT GCAGCCACCC AGATTTTCTT 1080
CTCACTTGGC CTGGGCTTCG GCAGCCTGAT CGCCTTCGCC AGCTACAATG AGCCATCCAA 1140
CAACTGCCAG AAGCACGCCA TCATCGTGTC CCTCATCAAC AGCTTCACCT CCATATTTGC 1200
CAGCATTGTC ACCTTCTCCA TCTATGGCTT CAAGGCCACC TTCAATTATG AAAACTGCTT 1260
GAAGAAGGTG AGTCTGCTGC TGACCAACAC TTTTGACCTT GAAGATGGCT TTTTGACAGC 1320
CAGCAACCTG GAGCAGGTGA AGGGCTACCT CGCATCTGCC TACCCAAGCA AATACAGCGA 1380

GATGTTCCCG CAAATCAAAA ACTGCAGCTT GGAATCGGAG CTAGACACGG CCGTCCAGGG 1440
CACTGGCCTG GCATTCATCG TCTACACAGA GGCCATTAAA AACATGGAGG TGTCCCAGCT 1500
GTGGTCGGTG CTCTACTTCT TCATGCTGCT GATGCTGGGC ATTGGGAGCA TGCTGGGGAA 1560
CACAGCGGCC ATCCTCACCC CTCTGACAGA CAGCAAGATC ATCTCCAGCC ACCTGCCCAA 1620
GGAGGCCATC TCAGGTCTGG TGTGCCTTGT CAACTGTGCC ATTGGCATGG TGTTCACGAT 1680
GGAGGCTGGG AACTACTGGT TTGACATATT CAACGACTAC GCGGCCACAC TGTCCCTGCT 1740
GCTCATCGTG CTGGTGGAGA CGATTGCCGT GTGCTACGTG TACGGGCTGA GGAGATTTGA 1800
AAGTGACCTT AAGGCCATGA CCGGCCGAGC TGTGAGCTGG TACTGGAAGG TGATGTGGGC 1860
TGGCGTAAGC CCACTGCTGA TTGTCAGCCT CTTTGTCTTC TACCTGAGCG ACTACATCCT 1920
CACGGGGACC CTGAAGTATC AAGCCTGGGA CGCCTCCCAG GGCCAGCTCG TGACCAAAGA 1980
TTACCCGGCC TATGCACTGG CTGTCATCGG GCTGCTTGTG GCCTCCTCCA CCATGTGCAT 2040
CCCCCTGGCG GCCCTGGGGA CTTTTGTTCA GCGTCGCCTC AAGAGGGGAG ACGCAGACCC 2100
CGTGGCCTGA GATGTGGGCT TCCCAGCCGC TCACGGTTTT ACAGATACTA TTTACAGGCG 2160
GAAACTCCTC GGCTGCTTTT TCAAATGCTT AAGCCAGGAG TGCTCAGCCC ATCAACTTCC 2220
TGAGTGTCTA AAGAAGATGA GGAAGGTGTG CAGGAAGAAA ACTCCCTTGG GAGAACGCAC 2280
ACCCTCCCGT GGTGGCTGTT CCTCCCTGTC ACCTGCCTCC TCATCATGGA AGGGGGTGGG 2340
CTATGAAAGC CGGTCTCAAA GATAACTGCA TCCTTCATTC CAGGAAAGCC CTAGAATTAG 2400
GGCACATTGC AAACTGAAAT ATGACTATAA TTCTTATGGG ACCAAATTTA AGCAATTTTT 2460
GTTTTTGGCT GAAGAGACAC CAAAATATTA GAGGACAAAT ATTTTTAGAT CCATTTAAGG 2520
AGTTTTGAAG TGCCTAAGAT GACCTATTTG TCAGTGGTGC AAAATTAATT CTCTTCTTTT 2580
TTGAGTTGTA GTGAATATGC AATTTCTGTG TTCCCCTTCC ACCCTTTAAA TCTTAGGATG 2640
ACAAGTTATA AAGAAAGAAG ATCTTTGTCT GGGACCCCCA AAGGGATCCT TTCTCTAAGG 2700
TCTCTGACGG TGGGTCCAGG ACCAGACCTC TCTACAAAAA ATTGCCCCAA CTACAGTTTG 2760
CAACCCCAAA CCACATTAGA ATCTGTGCAG ACATCCCTCC GTGGTGTGTG TCTTGGTGCA 2820
TTGGAAAAGG AGTCAGGAGC CACTGTGAGG TGAGAATGAA AGTGGATCTC AGCTGGGCAC 2880
GGTGGCTCAC GCCTGTAATC CTAGCACCTT GGGGGTCAAG GTGGGTGGAT CACTTGAGGT 2940
CAGGAGTTTG AGGCCAGCCT GGCCAACATG GCGAAACCCC ATTTCTACTA AAAATACAAA 3000
AAATTAGCTG GGAGTGGTGG CATAGGCCTG TAATTCCAGC TACTCTGGCT GCTGAGGCAC 3060
AAGAATCATT TGAACCTAGG AGGTGGCGGT TGCTGTGAGC CAAGATCATG CCACTCCACT 3120
CCAGTCTGGG CGACAGAGCA AGACTCTGAC TCAAAAAAAA AAAAAAAAA 3169

(2) INFORMATION FOR SEQ ID NO:2:

(i) SEQUENCE CHARACTERISTICS:
(A) LENGTH: 647 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Met Phe Ile Thr Val Leu Phe Ile Ala Gly Lys Thr Trp Lys Gln Pro
1               5                   10                  15
Arg Cys Ser Ser Gly Gly Glu Trp Ile Asn Lys Leu Trp His Ile Gln
            20                  25                  30
Thr Met Glu Cys Thr Glu Thr Ala Val Pro Asp Pro Arg Ala Gly Ala
        35                  40                  45
Glu Arg Ala Glu Ala Gly Ala Met Glu Lys Ala Arg Pro Leu Trp Ala
    50                  55                  60
Asn Ser Leu Gln Phe Val Phe Ala Cys Ile Ser Tyr Ala Val Gly Leu
65                  70                  75                  80
Gly Asn Val Trp Arg Phe Pro Tyr Leu Cys Gln Met Tyr Gly Gly Gly
                85                  90                  95
Ser Phe Leu Val Pro Tyr Ile Ile Met Leu Ile Val Glu Gly Met Pro
            100                 105                 110
Leu Leu Tyr Leu Glu Leu Ala Val Gly Gln Arg Met Arg Gln Gly Ser
        115                 120                 125
Ile Gly Ala Trp Arg Thr Ile Ser Pro Tyr Leu Ser Gly Val Gly Val
    130                 135                 140
Ala Ser Val Val Val Ser Phe Phe Leu Ser Met Tyr Tyr Asn Val Ile
145                 150                 155                 160
Asn Ala Trp Ala Phe Trp Tyr Leu Phe His Ser Phe Gln Asp Pro Leu
                165                 170                 175
Pro Trp Ser Val Cys Pro Leu Asn Gly Asn His Thr Gly Cys Asp Glu
            180                 185                 190
Glu Cys Glu Lys Ala Ser Ser Thr Gln Tyr Phe Trp Tyr Arg Lys Thr
        195                 200                 205
Leu Asn Ile Ser Pro Ser Leu Gln Glu Asn Gly Gly Val Gln Trp Glu
    210                 215                 220
Pro Ala Leu Cys Leu Leu Leu Ala Trp Leu Val Val Tyr Leu Cys Ile
225                 230                 235                 240
Leu Arg Gly Thr Glu Ser Thr Gly Lys Val Val Tyr Phe Thr Ala Ser
                245                 250                 255
Leu Pro Tyr Cys Val Leu Ile Ile Tyr Leu Ile Arg Gly Leu Thr Leu
            260                 265                 270
His Gly Ala Thr Asn Gly Leu Met Tyr Met Phe Thr Pro Lys Ile Glu
        275                 280                 285
Gln Leu Ala Asn Pro Lys Ala Trp Ile Asn Ala Ala Thr Gln Ile Phe
    290                 295                 300
Phe Ser Leu Gly Leu Gly Phe Gly Ser Leu Ile Ala Phe Ala Ser Tyr

305                 310                 315                 320
Asn Glu Pro Ser Asn Asn Cys Gln Lys His Ala Ile Ile Val Ser Leu
                325                 330                 335
Ile Asn Ser Phe Thr Ser Ile Phe Ala Ser Ile Val Thr Phe Ser Ile
            340                 345                 350
Tyr Gly Phe Lys Ala Thr Phe Asn Tyr Glu Asn Cys Leu Lys Lys Val
        355                 360                 365
Ser Leu Leu Leu Thr Asn Thr Phe Asp Leu Glu Asp Gly Phe Leu Thr
    370                 375                 380
Ala Ser Asn Leu Glu Gln Val Lys Gly Tyr Leu Ala Ser Ala Tyr Pro
385                 390                 395                 400
Ser Lys Tyr Ser Glu Met Phe Pro Gln Ile Lys Asn Cys Ser Leu Glu
                405                 410                 415
Ser Glu Leu Asp Thr Ala Val Gln Gly Thr Gly Leu Ala Phe Ile Val
            420                 425                 430
Tyr Thr Glu Ala Ile Lys Asn Met Glu Val Ser Gln Leu Trp Ser Val
        435                 440                 445
Leu Tyr Phe Phe Met Leu Leu Met Leu Gly Ile Gly Ser Met Leu Gly
    450                 455                 460
Asn Thr Ala Ala Ile Leu Thr Pro Leu Thr Asp Ser Lys Ile Ile Ser
465                 470                 475                 480
Ser His Leu Pro Lys Glu Ala Ile Ser Gly Leu Val Cys Leu Val Asn
                485                 490                 495
Cys Ala Ile Gly Met Val Phe Thr Met Glu Ala Gly Asn Tyr Trp Phe
            500                 505                 510
Asp Ile Phe Asn Asp Tyr Ala Ala Thr Leu Ser Leu Leu Leu Ile Val
        515                 520                 525
Leu Val Glu Thr Ile Ala Val Cys Tyr Val Tyr Gly Leu Arg Arg Phe
    530                 535                 540
Glu Ser Asp Leu Lys Ala Met Thr Gly Arg Ala Val Ser Trp Tyr Trp
545                 550                 555                 560
Lys Val Met Trp Ala Gly Val Ser Pro Leu Leu Ile Val Ser Leu Phe
                565                 570                 575
Val Phe Tyr Leu Ser Asp Tyr Ile Leu Thr Gly Thr Leu Lys Tyr Gln
            580                 585                 590
Ala Trp Asp Ala Ser Gln Gly Gln Leu Val Thr Lys Asp Tyr Pro Ala
        595                 600                 605
Tyr Ala Leu Ala Val Ile Gly Leu Leu Val Ala Ser Ser Thr Met Cys
    610                 615                 620
Ile Pro Leu Ala Ala Leu Gly Thr Phe Val Gln Arg Arg Leu Lys Arg
625                 630                 635                 640

Gly Asp Ala Asp Pro Val Ala
                645

(2) INFORMATION FOR SEQ ID NO:3:

(i) SEQUENCE CHARACTERISTICS:
(A) LENGTH: 719 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

GCTGGGCATT GGGAGCATGC TGGGGAACAC AGGNGCCATC CTCACCCCTC TGACAGACAG 60
CAAGATCATC TCCAGCCACC TGCCCAAGGA GGCCATCTCA GGTCTGGTGT GCCTTGTCAA 120
CTGTGCCATT GGCATGGTGT TCACGATGGA GGCTGGGAAC TACTGGTTTG ACATATTCAA 180
CGACTACGCG GCCACACTGT CCCTGCTGCT CATCGTGCTG GTGGAGACGA TTGCCGTGTG 240
CTACGTGTAC GGGCTGAGGA GATTTGAAAG TGACCTTAAG GCCATGACCG GCCGAGCTGT 300
GAGCTGGTAC TGGAAGGTGA TGTGGGCTGG CGTAAACCAC TGCATGATTG TAAGCCTCTT 360
TGTCTTCTAC CTGAGCGACT ACATCCTCAC GGGGACCCTG AAGTATCAAG CCTGGGACGC 420
CTCCCAGGGC CAGCTCGTGA CCAAAGATTA CCCGGCCTAT GCACTGGCTG TCATCGGGCT 480
GCTTGTGGCC TCCTCCACCA TGTGCATCCC CCTGGCGGCC CTGGGGACTT TTGTTCAGCG 540
TCGCCTCAAG AGGGGAGACG CAGACCCCGT GGCCTGAGAT GTGGGCTTCC CAGCCGCTCA 600
CGGTTTTACA GATACTATTT ACAGGCGGAA ACTCCTCGGC TGCTTTTTCA AATGCTTAAG 660
CCAGGAGTGC TCAGCCCATC AACTTCCTGA GTGTCTAAAG AAGATGAGGA AGGTGTGCA 719

(2) INFORMATION FOR SEQ ID NO:4:

(i) SEQUENCE CHARACTERISTICS:
(A) LENGTH: 19[?] amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Leu Gly Ile Gly Ser Met Leu Gly Asn Thr Gly Ala Ile Leu Thr Pro

1               5                   10                  15
Leu Thr Asp Ser Lys Ile Ile Ser Ser His Leu Pro Lys Glu Ala Ile
                20                  25                  30
Ser Gly Leu Val Cys Leu Val Asn Cys Ala Ile Gly Met Val Phe Thr
            35                  40                  45
Met Glu Ala Gly Asn Tyr Trp Phe Asp Ile Phe Asn Asp Tyr Ala Ala
        50                  55                  60
Thr Leu Ser Leu Leu Leu Ile Val Leu Val Glu Thr Ile Ala Val Cys
65                  70                  75                  80
Tyr Val Tyr Gly Leu Arg Arg Phe Glu Ser Asp Leu Lys Ala Met Thr
                85                  90                  95
Gly Arg Ala Val Ser Trp Tyr Trp Lys Val Met Trp Ala Gly Val Asn
            100                 105                 110
His Cys Met Ile Val Ser Leu Phe Val Phe Tyr Leu Ser Asp Tyr Ile
        115                 120                 125
Leu Thr Gly Thr Leu Lys Tyr Gln Ala Trp Asp Ala Ser Gln Gly Gln
    130                 135                 140
Leu Val Thr Lys Asp Tyr Pro Ala Tyr Ala Leu Ala Val Ile Gly Leu
145                 150                 155                 160
Leu Val Ala Ser Ser Thr Met Cys Ile Pro Leu Ala Ala Leu Gly Thr
                165                 170                 175
Phe Val Gln Arg Arg Leu Lys Arg Gly Asp Ala Asp Pro Val Ala
            180                 185                 190